# EUROPÄISCHE PATENTANMELDUNG

(11) **EP 4 714 934 A1**
(43) Veröffentlichungstag der Anmeldung: **25.03.2026**
(21) Anmeldenummer: 24201138.5
(22) Anmeldetag: 18.09.2024
(51) Int. Cl.: C07C 67/343, C07C 69/732

(54) **VERFAHREN ZUR HERSTELLUNG VON ALKYL-3-HYDROXY-2-METHYLENALKANOATEN**

(71) Anmelder: Bayer Aktiengesellschaft, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein neues Verfahren zur Herstellung von Alkyl-3-hydroxy-2-methylenalkanoaten der allgemeinen Formel (I).

## Beschreibung

Die vorliegende Erfindung betrifft ein neues Verfahren zur Herstellung von Alkyl-3-hydroxy-2-methylenalkanoaten der allgemeinen Formel **(I).**

Alkyl-3-hydroxy-2-methylenalkanoate der allgemeinen Formel **(I)** sind wichtige Vorstufen von agrochemischen Wirkstoffen (vgl. WO 2018/228985).

Im Stand der Technik sind zahlreiche Methoden zur Herstellung beschrieben, beispielsweise Chem. Rev 2010, 110, 5447-5674; J. Org. Chem 2003, 68, 692 - 700; J. Org. Chem 2010, 75, 8615 - 8626; J. Org. Chem 2001, 66, 5413 - 5418; Tetrahedron 2014, 70, 97 - 102; US 2004/0176243 A1.

Werden die Verbindungen der vorliegenden Erfindung nach einem der literaturbekannten Verfahren durchgeführt, resultieren Ausbeuten, die für eine großtechnische Synthese nicht ausreichend sind, oder es werden stöchiometrische Mengen von Hilfsstoffen eingesetzt in Kombination mit einer hohen Verdünnung durch Lösungsmittel oder Wasser. Das hat zur Folge, dass Reaktionszeiten von bis zu vier Tagen oder mehr benötigt werden. Auch das ist für eine großtechnische Synthese nicht vorteilhaft und verursacht durch die geringen Raum-Zeit-Ausbeuten u.a. hohe Kosten.

Der Erfindung lag somit die Aufgabe zugrunde, ein Verfahren zur Herstellung von Alkyl-3-hydroxy-2-methylenalkanoaten der allgemeinen Formel **(I)** zur Verfügung zu stellen, welches für die großtechnische Synthese geeignet ist sowie eine hohe Ausbeute in möglichst kurzer Zeit aufweist.

Die Aufgabe wurde erfindungsgemäß gelöst durch ein Verfahren zur Herstellung von Alkyl-3-hydroxy-2-methylenalkanoaten der allgemeinen Formel **(I)** 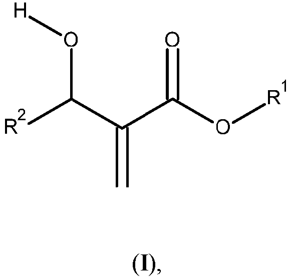 worin
R¹ H, C₁-C₆-Alkyl ist,
R² C₁-C₄-Alkyl ist,
dadurch gekennzeichnet, dass die Verbindungen der allgemeinen Formel **(II)** 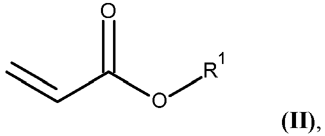
mit Verbindungen der allgemeinen Formel **(III)** 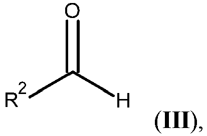
unter Zugabe von:
   - einem tertiären Amin-Katalysator und einem Alkohol der allgemeinen Formel **(IV)** R³OH oder
   - einem tertiären Amin-Katalysator und H₂O oder
   - einem tertiären Amin-Katalysator und einem Alkohol der allgemeinen Formel **(IV)** R³OH und H₂O reagieren,
   wobei
   - R¹ und R²: die oben genannten Bedeutungen haben und
   - R³: C₁-C₆-Alkyl ist.

   Bevorzugte Restedefinitionen für die Verbindungen der allgemeinen Formeln **(I), (II), (III)** und **(IV)** sind die folgenden:
      - R¹: ist Methyl, Ethyl, *i*-Propyl, *i*-Butyl,
      - R²: ist Methyl, Ethyl,
      - R³: ist C₁-C₄-Alkyl.
   Besonders bevorzugte Restedefinitionen für die Verbindungen der allgemeinen Formeln **(I), (II), (III)** und **(IV)** sind die folgenden:
      - R¹: ist Methyl, *i*-Propyl, *i*-Butyl,
      - R²: ist Methyl,
      - R³: ist C₁-C₄-Alkyl.
   Ganz besonders bevorzugte Restedefinitionen für die Verbindungen der allgemeinen Formeln **(I), (II), (III)** und **(IV)** sind die folgenden:
      - R¹: ist Methyl, *i*-Butyl,
      - R²: ist Methyl,
      - R³: ist C₁-C₄-Alkyl.
   Am stärksten bevorzugte Restedefinitionen für die Verbindungen der allgemeinen Formeln **(I), (II), (III)** und **(IV)** sind die folgenden:
      - R¹: ist Methyl,
      - R²: ist Methyl,
      - R³: ist Methyl.

### Erläuterung der Verfahren und Zwischenprodukt

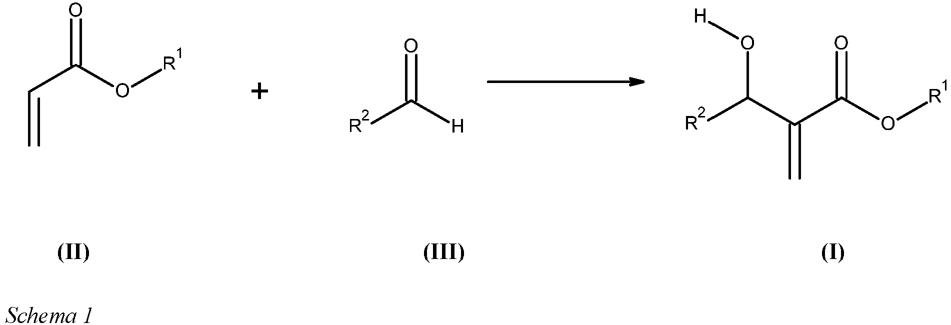

Die Aufgabe wurde gelöst durch ein Verfahren zur Herstellung von Alkyl-3-hydroxy-2-methylenalkanoaten der allgemeinen Formel **(I)** dadurch gekennzeichnet, dass die Verbindungen der allgemeinen Formel **(II)** mit Verbindungen der allgemeinen Formel **(III)** unter Zugabe von:
- einem tertiären Amin-Katalysator und einem Alkohol der allgemeinen Formel **(IV)** R³OH oder
- einem tertiären Amin-Katalysator und H₂O oder
- einem tertiären Amin-Katalysator und einem Alkohol der allgemeinen Formel **(IV)** R³OH und H₂O
   reagieren, wobei
   R¹ und R² die oben genannten Bedeutungen haben und
   R³ C₁-C₆-Alkyl ist.

Die Verbindungen der Formel **(II)** und **(III)** sind aus der Literatur bekannt, z.B. aus den in der Einleitung zitierten Referenzen.

Als tertiäre Amin-Katalysatoren können beispielhaft verwendet werden: Triethylendiamin (DABCO), 3-Quinuclidinol (3-Hydroxyquinuclidin), Quinuclidin, PPh₃, DBU sowie Kombinationen aus diesen Amin-Katalysatoren.

Bevorzugt ist die Verwendung von Triethylendiamin, Quinuclidin und 3-Quinuclidinol oder auch Mischungen davon. Besonders bevorzugt ist die Verwendung von Triethylendiamin.

Bevorzugt ist der Einsatz von 0,1 bis 0,4 Äquivalenten (Äq) des tertiären Amin-Katalysators bezogen auf 1 Äquivalent der Verbindungen der allgemeinen Formel **(II).**

Besonders bevorzugt ist der Einsatz von 0,15 bis 0,25 Äquivalenten des tertiären Amin-Katalysators bezogen auf 1 Äquivalent der Verbindungen der allgemeinen Formel (**II**).

Das Verhältnis der Verbindungen der allgemeinen Formel (**III**) und **(II)** zueinander kann variieren. Bevorzugt werden 1 bis 2 Äquivalente der Verbindungen der allgemeinen Formel (**III**) bezogen auf 1 Äquivalent der Verbindungen der allgemeinen Formel (**II**) verwendet, besonders bevorzugt 1 bis 1,5 Äquivalente der Verbindungen der Formel (**III**) bezogen auf 1 Äquivalent der Verbindungen der allgemeinen Formel (II), ganz besonders bevorzugt 1 bis 1.3 Äquivalente der Verbindungen der Formel allgemeinen (**III**) bezogen auf 1 Äquivalent der Verbindungen der allgemeinen Formel (**II**).

Die Zyklisierung wird gewöhnlich in einem Temperaturbereich von 0°C bis 70°C, vorzugsweise 10°C bis 50 °C durchgeführt.

Des Weiteren wird die Zyklisierung in Gegenwart eines Lösungs- oder Verdünnungsmittels oder einer Kombination dieser durchgeführt, nämlich einem Alkohol der allgemeinen Formel **(IV)** R³OH und/oder Wasser. Bevorzugt wird Methanol und/oder Wasser eingesetzt. Ganz besonders bevorzugt ist die Kombination aus Methanol und Wasser.

Bei einer Kombination aus Alkohol der allgemeinen Formel **(IV)** R³OH und Wasser ist ein breites Spektrum von Verhältnissen zwischen Wasser und Alkohol möglich. Bevorzugt werden Mischungen aus Wasser und einem Alkohol der allgemeinen Formel **(IV)** R³OH in Verhältnissen zwischen 100:1 und 1:100 ausgewählt, besonders bevorzugt zwischen 10:1 und 1:10 und am stärksten bevorzugt zwischen 3:1 und 1:3 bezogen auf das Verhältnis von Wasser zu Alkohol eingesetzt.

Ganz besonders bevorzugt ist die Kombination aus Wasser und Methanol in Verhältnissen zwischen 3:1 und 1:3 bezogen auf das Verhältnis von Wasser zu Alkohol eingesetzt

### Beispiele

Die vorliegende Erfindung wird anhand der nachfolgenden Beispiele näher erläutert, ohne die Erfindung dabei auf diese einzuschränken.

### Messverfahren

Die Produkte wurden mittels ¹H-NMR Spektroskopie und/oder LC/MS (Liquid Chromatography Mass Spectrometry) charakterisiert.

Die NMR-Spektren wurden mit einem Bruker Avance 400, ausgestattet mit einem Durchflussprobenkopf (60 µl Volumen), bestimmt. In Einzelfällen wurden die NMR Spektren mit einem Bruker Avance II 600 gemessen.

### Beispiel 1: Synthese von rac-Methyl-3-hydroxy-2-methylene-butanoat (I-1)

59,8 g (0,51 mol, 0,18 eq) 1,4-Diazabicyclo[2.2.2]octan wurden in 46,5 g Methanol gelöst und mit 250 g (2,87 mol, 1,0 eq) Methylacrylat versetzt. Bei 15-20 °C wurde innerhalb von 15 min eine auf 5 °C gekühlte Lösung von 152,7 g (3,45 mol, 1,2 eq) Acetaldehyd in 24,6 g Wasser zudosiert und die Reaktion nach vollständiger Dosierung für 24 h bei 30 °C gerührt. Nach destillativer Entfernung des Methanols wurde mit 160 g 20% Salzsäure ein pH-Wert von 3 eingestellt und die Lösung dreimal mit jeweils 100 mL tert-Butylmethylether extrahiert. Die gesammelten organischen Extrakte wurden vereinigt, einmal mit 100g wässriger Natriumhydrogencarbonat-Lösung gewaschen und das Lösungsmittel destillativ entfernt. Nach azeotroper Trocknung des Rückstands mit Toluol wurden 325,1 g (94,9 Gew.%, 82% Ausbeute) als farblose Flüssigkeit erhalten.

¹H-NMR (400 MHz, CDCl₃): 6,22 (m, 1H), 5,84 (m, 1H), 4,62 (quint. 1H), 3,79 (s, 3H), 2,74 (br s, 1H) 1.39 (s, 3H) ppm.

### Beispiel 2: Herstellung von rac-Methyl 3-hydroxy-2-methylene-butanoat (I-1)

In einem 1L Reaktor unter Stickstoff wurden 24,6 g Wasser (0,48 Äq.) und 46,5 g Methanol (0,5 Äq.) bei 20 °C vorgelegt und darin 59.8 g Triethylendiamin (DABCO, 0,18 Äq) gelöst. Anschließend wurden 115,0 g Methylacrylat (1,33 mol. 0,46 Äq.) zugegeben sowie im Anschluss bei 10-20 °C über 1 h eine Mischung von 135,0 g Methylacrylat (1,55 mol, 0,54 Äq.) und 152,7 g Acetaldehyd (1,2 Äq.) zudosiert. Die Mischung wurde dann auf 30 °C erwärmt und bei dieser Temperatur 24 h gerührt. Im Anschluss wurde die Lösung auf 0-5 °C gekühlt und mit 200,0 g einer 30 Gew.%igen wässrigen Schwefelsäure versetzt und ein pH von 1 eingestellt. Die Phasen wurden getrennt, die untere wässrige Phase dreimal mit je 100 mL tert-Butylmethylether (MTBE) extrahiert und die vereinigten organischen Phasen dann mit einer 20 Gew.%igen wässrigen Kaliumhydrogencarbonat Lösung bei pH = 7 gewaschen. Nach Phasentrennung sowie destillativer Entfernung des Lösungsmittels sowie Wasser wurde das Produkt als farbloses Öl isoliert: 323,3 g (95,4 Gew.%, Ausbeute 82%).

### Beispiel 3: Herstellung von rac-Methyl 3-hydroxy-2-methylene-butanoat (I-1)

In einem 1L Reaktor unter Stickstoff wurden 93,1 g Methanol (1,0 Äq.) bei 20 °C vorgelegt und darin 59,8 g Triethylendiamin (DABCO, 0,18 Äq) gelöst. Anschließend wurden 250,0 g Methylacrylat (2,87 mol. 1,0 Äq.) zugegeben sowie im Anschluss bei 0 °C eine Mischung von 152,7 g Acetaldehyd (1,2 Äq.) in 49,2 g Wasser (0,95 Äq.) in 1h zudosiert. Die Mischung wurde dann auf 30 °C erwärmt und bei dieser Temperatur 24 h gerührt. Im Anschluss bei 20 °C mit 167.0 einer 20 Gew.%igen wässrigen Salzsäure ein pH von 3 eingestellt und die wässrige Phase dreimal mit je 100 mL tert-Butylmethylether (MTBE) extrahiert und die vereinigten organischen Phasen dann mit einer 8 Gew.%igen wässrigen Natriumhydrogencarbonat Lösung bei pH = 7 gewaschen. Nach Phasentrennung sowie destillativer Entfernung des Lösungsmittels sowie Wasser wurde das Produkt als farbloses Öl isoliert: 338,6 g (88,6 Gew.%, Ausbeute 80%).

### Beispiel 4: Herstellung von rac-Isobutyl 3-hydroxy-2-methylene-butanoate (I-2)

In einem 500mL Reaktor unter Stickstoff wurde 15,5 g Triethylendiamin (DABCO, 0,20 Äq) vorgelegt. Anschließend wurden 90 g Isobutylylacrylat (686,7 mmol, 1,0 Äq.), 31 g Butanol (0,60 Äq), 3.7 g Wasser (0,30 Äq.) der Reihe nach zugegeben sowie im Anschluss in einem Wasserbad 10-20 °C gekühlt. Anschließend wurde 40 g Acetaldehyd (1,3 Äq.) über 20 min zudosiert. Die Mischung wurde dann auf 40 °C erwärmt und bei dieser Temperatur 3 Tage gerührt.

Im Anschluss wurde die Lösung auf Raumtemperatur gekühlt und mit 200 mL tert-Butylmethylether (MTBE) sowie 150 mL 2M HCl versetzt und ein pH von 1 eingestellt. Die Phasen wurden getrennt, die untere wässrige Phase zweimal mit je 200 mL (MTBE) extrahiert. Nach Phasentrennung sowie destillativer Entfernung des Lösungsmittels sowie Wasser wurde das Produkt als farbloses Öl isoliert: 107 g (96 Gew.%, Ausbeute 87%).

**¹H NMR (600 MHz, CDCl₃) δ (ppm):** 6.20 (s, 1H), 5.80 (s, 1H), 4.60 q, *J =* 6.5 Hz, 1H), 3.95 (d, *J =* 6.6 Hz, 2H), 2.85 (s, 1H), 2.03 - 1.92 (m, 1H), 1.37 (d, *J =* 6.5 Hz, 3H), 0.94 (d, *J =* 6.8 Hz, 6H).

**¹³C (151 MHz, CDCl₃) δ (ppm):** 166.77, 143.87, 123.94, 70.99, 67.34, 27.85, 22.21, 19.22.

**Tabelle 1**

| Umsatz nach HPLC nach 24 h 20 °C | | | | | |
|---|---|---|---|---|---|
| Nr. | tertiäre Amin-Katalysatoren (mol%) | Additive 1 (Äq.) | Additive 2 (Äq.) | Methylacrylat (%) | Produkt (%) |
| 1 | DABCO (20) | - | - | 64 | 36 |

**Tabelle 2**

| Reaktionen mit 1,1 Äq Acetaldehyd. Umsatz nach HPLC nach 18 h 20 °C | | | | | |
|---|---|---|---|---|---|
| Nr. | tertiäre Amin-Katalysatoren (mol%) | Additive 1 (Äq.) | Additive 2 (Äq.) | Methylacrylat (%) | Produkt (%) |
| 25^{a)} | DABCO (18) | MeOH (1,0) | H₂O (0,5) | 8 | 92 |
| 26^{a)} | DABCO (18) | MeOH (0,5) | H₂O (1,0) | 5 | 95 |
| 27^{a)} | DABCO (18) | MeOH (0,25) | H₂O (0,5) | 5 | 95 |
| 28 ^{a,b)} | DABCO (10) | MeOH (0,4) | - | 31 | 67 |
| 29^{a,b)} | DABCO (10) | MeOH (0,6) | - | 31 | 68 |
| 30^{a,b)} | DABCO (20) | MeOH (0,4) | - | 29 | 70 |
| 31 ^{a,b)} | DABCO (20) | MeOH (0,6) | - | 31 | 68 |
| 32^{a,b)} | DABCO (10) | - | H₂O (0,5) | 18 | 76 |
| 33^{a,b)} | DABCO (10) | - | H₂O (1,0) | 29 | 64 |
| 34^{a,b)} | DABCO (20) | - | H₂O (0,5) | 3 | 90 |
| 35 ^{a,b)} | DABCO (20) | - | H₂O (1,0) | 3 | 88 |
| 36 ^{a,b)} | DABCO (10) | MeOH (0,5) | H₂O (0,5) | 16 | 82 |
| 37 ^{a,b)} | DABCO (10) | MeOH (0,5) | H₂O (1,0) | 17 | 80 |
| 38 ^{a,b)} | DABCO (20) | MeOH (0,5) | H₂O (0,5) | 6 | 92 |
| 39 ^{a,b)} | DABCO (20) | MeOH (0,5) | H₂O (1,0) | 5 | 91 |
| | | | | | |
| a) Umsatz nach 24 h. b) Reaktionen mit 1,2 Äq Acetaldehyd. | | | | | |

**Tabelle 3**

| Reaktionen mit 20 mol% DABCO, 40 °C, 1,3 Äq Acetaldehyd. Umsatz nach HPLC | | | | | | |
|---|---|---|---|---|---|---|
| **Nr.** | **R¹** | **Additive 1 (0.60 Äq.)** | **Additiv 2 (0.30 Äq.)** | **Acrylat (%)** | **Produkt (%)** | **Zeit** |
| 1 | i-PrO | i-PrOH | H₂O | 7 | 93 | 2d |
| 2 | i-PrO | i-PrOH | H₂O | 4 | 95 | 3d |
| 3 | i-PrO | i-PrOH | - | 16 | 80 | 2d |
| 4 | i-PrO | i-PrOH | - | 9 | 88 | 3d |
| 5 | i-PrO | - | H₂O | 7 | 92 | 2d |
| 6 | i-PrO | - | H₂O | 5 | 91 | 3d |
| 7 | i-PrO | - | - | 23 | 76 | 2d |
| 8 | i-PrO | - | - | 11 | 88 | 3d |
| 9 | i-BuO | i-BuOH | H₂O | 5 | 93 | 2d |
| 10 | i-BuO | i-BuOH | - | 11 | 87 | 2d |
| 11 | i-BuO | - | H₂O | 5 | 92 | 2d |
| 12 | i-BuO | - | - | 19 | 79 | 2d |
| 13 | t-BuO | t-BuOH | H₂O | 28 | 71 | 2d |
| 14 | t-BuO | t-BuOH | H₂O | 21 | 78 | 3d |
| 15 | t-BuO | t-BuOH | - | 50 | 49 | 2d |
| 16 | t-BuO | t-BuOH | - | 37 | 60 | 3d |
| 17 | t-BuO | - | H₂O | 30 | 69 | 2d |
| 18 | t-BuO | - | H₂O | 24 | 73 | 3d |
| 19 | t-BuO | - | - | 76 | 23 | 2d |
| 20 | t-BuO | - | - | 57 | 40 | 3d |

## Patentansprüche

1. Verfahren zur Herstellung von Alkyl-3-hydroxy-2-methylenalkanoaten der allgemeinen Formel (I) 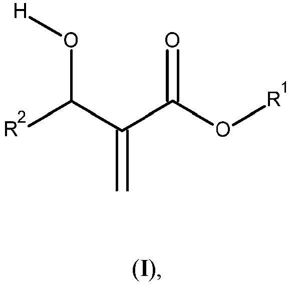 worin
R¹ H, C₁-C₆-Alkyl ist,
R² C₁-C₄-Alkyl ist,
**dadurch gekennzeichnet, dass** die Verbindungen der allgemeinen Formel **(II)** 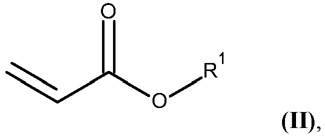 mit Verbindungen der allgemeinen Formel (III) 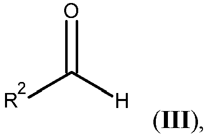 unter Zugabe von:
- einem tertiären Amin-Katalysator und einem Alkohol der allgemeinen Formel **(IV)** R³OH oder
- einem tertiären Amin-Katalysator und H₂O oder
- einem tertiären Amin-Katalysator und einem Alkohol der allgemeinen Formel **(IV)** R³OH und H₂O reagieren,
wobei
R¹ und R² die oben genannten Bedeutungen haben und
R³ C₁-C₆-Alkyl ist.

2. Verfahren nach Anspruch 1, wobei die Restedefinitionen für die Verbindungen der allgemeinen Formeln **(I), (II), (III)** und **(IV)** die folgenden sind:
R¹ ist Methyl, Ethyl, i-Propyl, i-Butyl,
R² ist Methyl, Ethyl,
R³ ist C₁-C₄-Alkyl.

3. Verfahren nach einem der Ansprüche 1 bis 2, wobei die Restedefinitionen für die Verbindungen der allgemeinen Formeln **(I), (II), (III)** und **(IV)** die folgenden sind:
R¹ ist Methyl, i-Propyl, i-Butyl,
R² ist Methyl,
R³ ist C₁-C₄-Alkyl.

4. Verfahren nach einem der Ansprüche 1 bis 3, wobei die Restedefinitionen für die Verbindungen der allgemeinen Formeln **(I), (II), (III)** und **(IV)** die folgenden sind:
R¹ ist Methyl, i-Butyl,
R² ist Methyl,
R³ ist C₁-C₄-Alkyl.

5. Verfahren nach einem der Ansprüche 1 bis 4, wobei die Restedefinitionen für die Verbindungen der allgemeinen Formeln **(I), (II), (III)** und **(IV)** die folgenden sind:
R¹ ist Methyl,
R² ist Methyl,
R³ ist Methyl.

6. Verfahren nach einem der Ansprüche 1 bis 5, **dadurch gekennzeichnet, dass** 0,1 bis 0,4 Äquivalente tertiärer Amin-Katalysator bezogen auf 1 Äquivalent der Verbindung der allgemeinen Formel (**II**) verwendet werden.

7. Verfahren nach einem der Ansprüche 1 bis 5, **dadurch gekennzeichnet**, 0,15 bis 0,25 Äquivalente tertiärer Amin-Katalysator bezogen auf 1 Äquivalent der Verbindung der allgemeinen Formel (**II**) verwendet werden.

8. Verfahren nach einem der Ansprüche 1 bis 7, **dadurch gekennzeichnet, dass** das Lösungsmittel Methanol und/oder Wasser ist.

9. Verfahren nach einem der Ansprüche 1 bis 7, **dadurch gekennzeichnet, dass** das Lösungsmittel eine Kombination aus Methanol und Wasser ist.

10. Verfahren nach einem der Ansprüche 1 bis 7, **dadurch gekennzeichnet, dass** das Lösungsmittel eine Kombination aus Wasser und Methanol ist und in Verhältnissen zwischen 3:1 und 1:3 bezogen auf das Verhältnis von Wasser zu Alkohol eingesetzt wird.

11. Verfahren nach einem der Ansprüche 1 bis 7, **dadurch gekennzeichnet, dass** das Lösungsmittel eine Kombination aus Wasser und Methanol ist und in Verhältnissen zwischen 3:1 und 1:3 bezogen auf das Verhältnis von Wasser zu Alkohol eingesetzt wird, wobei der tertiäre Amin-Katalysator DABCO ist.

12. Verfahren nach einem der Ansprüche 1 bis 11, **dadurch gekennzeichnet, dass** die Reaktion bei 10°C bis 50 °C durchgeführt wird.

13. Verfahren nach einem der Ansprüche 1 bis 7, **dadurch gekennzeichnet, dass** der tertiäre Amin-Katalysator DABCO ist.
